Europäisches Patentamt

European Patent Office

Office européen des brevets

(11) Publication number: **0 223 762 B1**

(12) **EUROPEAN PATENT SPECIFICATION**

(45) Date of publication of patent specification: **15.04.92** (51) Int. Cl.⁵: **A61K 33/42**

(21) Application number: **86850396.2**

(22) Date of filing: **18.11.86**

(54) **Use of a composition for the manufacture of a medicament for the improvement of cellular metabolism.**

(30) Priority: **22.11.85 SE 8505527**

(43) Date of publication of application:
**27.05.87 Bulletin 87/22**

(45) Publication of the grant of the patent:
**15.04.92 Bulletin 92/16**

(84) Designated Contracting States:
**AT BE CH DE ES FR GB IT LI LU NL SE**

(56) References cited:
**FR-A- 2 325 330**
**US-A- 4 322 407**

(73) Proprietor: **Firma Prevent**
**Sanekullavägen 35**
**S-217 74 Malmö(SE)**

Proprietor: **MEDINA HB**
**Vikingagatan 44**
**S-216 18 Malmö(SE)**

(72) Inventor: **Henningsen, Nels**
**Sanekullavägen 35**
**S-217 74 Malmö(SE)**
Inventor: **Jaedig, Steen**
**Vikingagatan 44**
**S-216 18 Malmö(SE)**

(74) Representative: **Barnieske, Hans Wolfgang**
**c/o H.W. Barnieske Patentbyrä AB S:ta Ragn-**
**hildsgatan 24-26 P.O. Box 25**
**S-151 21 Södertälje 1(SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**Description**

The present invention relates to the use of a composition to improve the cellular metabolism in human-beings with adequate calorie consumption in combination with a salt-deficient diet.

There is increasing evidence that the cellular metabolism of people living in our modern society does not function satisfactorily. The extremely high consumption of sodium chloride plays a decisive role in the development of metabolic deviations causing conditions such as high blood pressure, diabetes type II and obesity frequently occuring nowadays. Bone brittleness, vascular spasms and arteriosclerosis can also be traced back to deteriorated cell metabolism. This is described in more detail inter alia in an article in Klin Wochenschr. (1985) 63 (Suppl. III):4-8 "The Sodium Pump and Energy Regulation", N. C. Henningsen.

Studies have been carried out on dogs indicating that ordinary cooking salt increases excretion of, i.e. leaches out, potassium, phosphate, magnesium and calcium in the urine and clinical experiments show that the chloride ion competes with the phosphate ion, see Massry S G, Friedler R M, Coburn J W, "Excretion of phosphate and calcium", Arch Int. Med. 131: 828-59, 1973.

Experiments with rats, carried out over an extended period, also showed that salt intake, as well as energy intake, was directly and significantly correlated to the age-related fat mass, see Kidney International, Vol, 27 (1985) pages 497-502, "Effect of variations in dietary sodium intake ....", Brensilver et al.

The problem of obesity is now receiving considerable attention and vast numbers of slimming methods are appearing on the market. However, many of these are based on prejudices and also leave the person with very poor chances of maintaining his ideal weight, partly due to an incorrect nitrogen balance existing in combination with various deficiencies. Practically without exception, this leads to a return to the original weight within a year or two and in many cases the new weight is even higher than before slimming.

Physical training has also been and is still considered a wonder cure in the fight against overweight. However, careful investigation shows that people suffering from obesity tend to increase rather than decrease in weight after a period of physical training, see Mandroukas K, Krotkiewski M, et al, "Physical training in obese women", Eur J Appl Physiol (1984) 52: pages 355-361. Physical training while maintaining the same diet is nowadays considered in medical circles to be an ineffectual method of achieving a weight reduction.

Slimming by means of a drastic reduction in calorie intake to 500 kcal/day, for instance, requires replacement of the loss of essential fatty acids, amino acids, minerals and vitamins and to achieve a permanent result, it must be followed by a radical change of diet.

A combination of strenuous physical training and rigorous slimming often results in a negative nitrogen balance and requires extra amino acids and a change of diet. The current method for athletes now is a special diet to suit the type of work they are to perform.

Studies have also been performed indicating that the ability to generate heat after a test meal is poorer in obese people than in lean people. These studies show that obese people often eat less than lean people but the body is incapable of disposing of the energy and it is instead stored as fat, see J. Clin. Invest. Volume 76 (September 1985) pages 1107 - 1112, Segal et al., "Thermic Effect of Food in Lean and Obese Men" and J. Clin Nuctr. 42 (August 1985) pages 177-181, Swaminathan et al., "Thermic effect of feeding carbohydrate, fat, protein and mixed meal in lean and obese subjects".

To summarize, therefore, it can be established that a new philosophy is now emerging: slimming is combined with mineral and vitamin supplements, which are not supplied in sufficient quantities in the normal diet. Those diseases which may be summarily termed our welfare diseases are not considered as a whole.

The object of the present invention is the use of a composition which improves the cellular metabolism and thus prevents the occurrence of conditions such as high blood pressure, gross obesity, diabetes type II, and which enables the body itself to take care of excess energy supplied by the diet in some other way than by storing it in fatty tissue, entailing only minor dietary adjustment. According to the invention the composition is also suitable for use as drinks for athletes, as well as prophylactically.

The intracellular absorption of potassium in an active process works by way of the "sodium/potassium pump". In nature only pairs of ions with positive and negative charge are permitted, and potassium must therefore be accompanied by a negative ion. The dominant ion intracellularly is phosphate which, contrary to the extracellular negative ion, chloride, also plays a dominant part in building up the cell energy (ATP and creatine phosphate). If potassium is to be retained to a sufficient extent in the cell, it must be accompanied by phosphate. Previously, potassium chloride has always been administered, which has reduced the ability of the potassium to remain in the cell. Instead, a large quantity of the potassium chloride supplied to the body, was excreted through the kidneys which always react to an excess of potassium chloride by increased excretion, see Klin Wochenschr. (1985) 63 (Suppl. III): 4-8, "The Sodium Pump and Energy Regulation", N C Henningsen.

Improved cellular metabolism in human beings with adequate calorie consumption in combination with a salt-deficient diet is achieved with the use of a composition for the manufacture of a medicament which comprises potassium, magnesium and hydrogen phosphate or dihydrogen phosphate ions in an amount of 30-80 mmol $K^+$, 0-20 mmol $Mg^{2+}$ and 15-50 mmol $H_2PO_4^{2-}$ or $H_2PO_4^{1-}$. It has surprisingly been found that the supply of excess potassium, magnesium and phosphate, preferably in combination with a diet low in salt, results in improved metabolism due to better oxygen absorption and an increased ability to produce heat. A contributory cause of this effect is the fact that the contents, particularly of phosphate and potassium, in the blood are lower than is physiologically normal. This in turn is caused by the leaching effect of our high salt intake on potassium and phosphate. The composition shall be taken in overdoses and at least up to 200 - 400 % more than is normally excreted in the urine per hour, can be taken by otherwise healthy persons.

The compounds included in the composition preferably consist of potassiumdihydro-phosphate and magnesiumhydro-phosphate. It has been found that, unlike potassium chloride, potassium phosphate retains both magnesium and calcium.

Supplying the body with potassium, magnesium and phosphate in excess creates a new balance in the body, since all cells of the body will be passed by liquid having high contents of potassium,magnesium and phosphate, thus inter alia recreating the body's correct sodiumpotassium balance and thus also its energy balance.

According to a preferred embodiment of the invention the composition also includes carbohydrate. The addition of carbohydrate activates insulin which facilitates absorption of glucose and thus also of minerals, in the cells.

According to another embodiment of the invention, the composition contains 50 mmol $K^+$, 10 mmol $Mg^{2+}$ and 30 or 35 mmol $HPO_4^{2-}$ or $H_2PO_4^{1-}$ or mixtures thereof.

According to yet another embodiment of the invention the composition includes carbohydrate in the form of cane-sugar, glucose and/or fructose.

The composition according to the invention may exist in the form of a powder to be dissolved in liquid or mixed with some other food.

The composition according to the invention may also exist in the form of tablets to be dissolved in liquid, chewed or swallowed whole or in the form of a suspension or capsules.

If the composition according to the invention is taken together with meals, the quantity of carbohydrate necessary to activate the insulin production to the desired extent is normally obtained. However, if the composition is taken separately, it should include carbohydrate in the form of about 3 - 8 g glucose (daily dose), for instance, or the composition may be taken together with carbohydrate in suitable form.

Practically no negative effects have been found in healthy people consuming the composition according to the invention and in order to further illustrate the invention an account is given in the following of a number of clinical experiments performed.

I. The acute effect of potassium phosphate, potassium cloride and placebo on the electrolyte balance of helthy control subjects.

Earlier studies of animals and human-beings have indicated an effect with respect to potassium, calcium and phosphate, caused by sodium chloride intake (cooking salt). On 10 healthy control subjects with an average age of 25 years (5 women and 5 men), Henningsen N C, Jaedig S, Skinhøj A, show in "Is potassium phosphate the potassium substitute of choice?" (to be published in 1986), using a controlled, double-blind, cross-over method, that great differences exist with respect to electrolyte excretion in the urine after consuming either potassium phosphate (dihydro potassium phosphate), potassium chloride or a placebo. In the two first cases the control subjects received 50 mmol potassium and 25 mmol phosphate or 50 mmol chloride, respectively. The blood concentration and urine concentration of sodium potassium, chloride, magnesium and calcium were measured after 0, 2, 4 and 24 hours and in the urine from the whole 24-hour period. The potassium chloride immediately increased the excretion of both sodium and chloride for up to 4 hours. The excretion of chloride was of course expected, but the excretion of phosphate also increased significantly. The phosphate and magnesium contents in the blood dropped significantly. After consumption of potassium phosphate a distinct increase of the sodium and chloride contents in the urine was observed during the first 2 hours, as well as a distinct increase of phosphate as expected. Over the remaining 24-hour period a clear tendency to reduced excretion of both magnesium and calcium was observed with potassium phosphate. These differences are highly signigicant when compared with the placebo and with potassium chloride. An electrolyte mixture which can in this way retain magnesium and calcium as well as potassium and phosphate in the body might be imagined to affect the cellular metabolism to a great extent. This is now being tested in several studies. The high sodium chloride content in the average diet can therefore obviously be expected to be one of the prime causes of the tissue concentration of potassium and phosphate showing a marked reduction with age in people living in the western civilisation as compared with primitive people and animals existing on a diet deficient in salt, see The New England Journal of Medicine, vol. 312 No. 5, pages 283 - 289, "Paleolithic Nutrition", S Boyd Eaton, M.D. and Melvin Konner, Ph.D., "A Consideration of Its Nature and Current Implications", and Klin Wochenschr. (1985) 63 (Suppl. III):4-8 "The Sodium Pump and Energy Regulation", N.C. Henningsen.

II. Increased oxygen absorption or cell metabolism in obese women after consuming juice with the addition of potassium-magnesium phosphate.

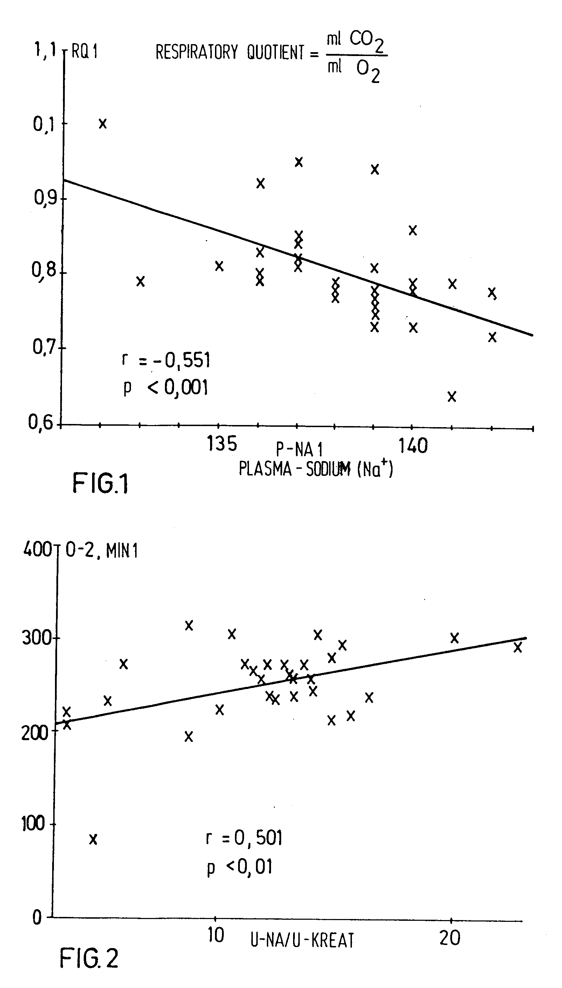

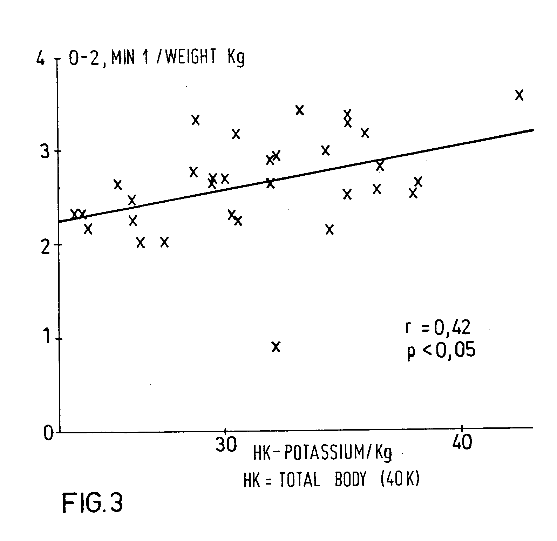

33 grossly obese women (at least 30% overweight) with an average age of 44 years were examined in an acute study of their basal metabolism by way of oxygen absorption and carbon dioxide production. After 14 hours of fasting and 30 minutes rest in the morning, a significant negative correlation was noted between the respiratory quotient

$$(RQ = \frac{CO_2 vol.}{O_2 vol.})$$

and plasma-sodium (Fig. 1). There was also a significant positive correlation between the salt excretion and oxygen absorption of the preceding 24-hour period (Fig. 2). Finally, there was also a significant correlation between the potassium throughout the body per kilo of body weight and the quantity of oxygen absorbed per minute (Fig. 3). This means that besides affecting the individual nutritional groups (fat, carbohydrate and protein), the salt intake also has a relatively strong effect on our cell metabolism in an unfavourable anaerobic direction - (reduced oxidative metabolism or $CO_2$ production per volume of oxygen absorbed).

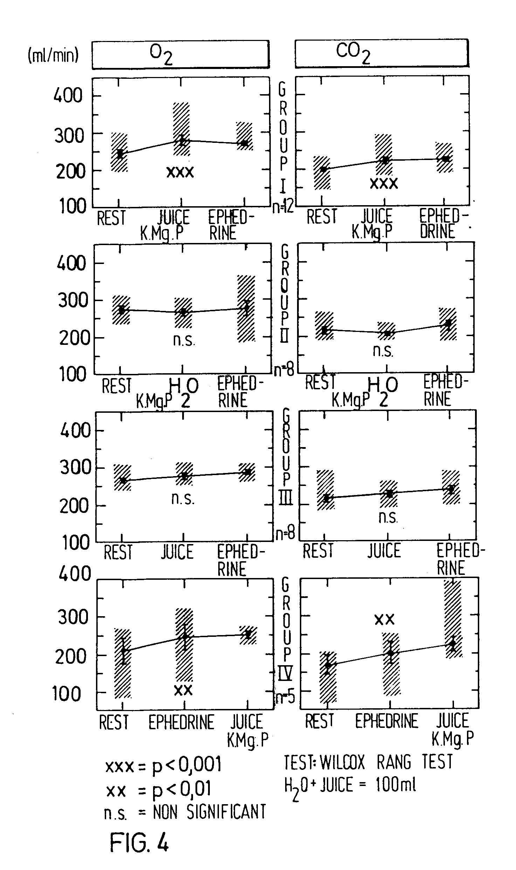

The patients were divided into four groups. One group was then given juice with potassium-magnesium phosphate, another group was given water with the same quantity of potassium-magnesium phosphate, one group was given juice without minerals and the last group was given an initial injection of Efedrinett, a composition known to increase the metabolic rate. The group of 12 women who received the potassium-magnesium phosphate in juice also exhibited a marked increase in oxygen absorption after 20 minutes (+14.1%), see Fig. 4. This was also followed by an increased potassium and phosphate content in the blood, as well as blood sugar and insulin. The two groups with either water and minerals or juice exhibited no significant change in oxygen absorption although altered mineral content or insulin and blood sugar content were observed in each group. The group given Efedrin exhibited a 15% increase in oxygen absorption, as expected, with a simultaneous increase in blood sugar and insulin. A reduction in the potassium and phosphate contents in the blood was also observed in this group. The conclusion is that with a high content of intracellular electrolytes, calories such as carbohydrates in this case, greatly increase the cell metabolism due to a combined effect via insulin. Since the modern diet is extremely rich in salt and relatively poor in the above-mentioned, primarily intracellular minerals, a chronic gradual decrease may be expected in the metabolic rate of human-beings. This has also been found to be the case in experiments performed since the war. Modern slimming diets, with possible calorie reduction, must therefore contain a significant excess of the above-mentioned minerals which per se do not entail any risks. The diet of primitive man and today's animal diet was and is clearly enriched with potassium, magnesium and phosphate and a low salt content, see The New England Journal of Medicine, vol. 312 No. 5, pages 283 - 289, "Paleolithic Nutrition", S Boyd Eaton, M.D. and Melvin Konner, Ph.D., "A Consideration of Its Nature and Current Implications", and Klin Wochenschr. (1985) 63 (Suppl. III): 4-8 "The Sodium Pump and Energy Regulation", N. C. Henningsen.

III. Long-term experiment with either potassium, magnesium phosphate, Glucophag, Efedrin or placebo to slim grossly obese women.

All together 33 women, divided into four groups, participated in an experiment in which one group received a placebo and advice to reduce their calorie intake to 1200. The other three groups were restricted to 1200 - 1600 calories and a salt-deficient diet. One group received an extra supplement of potassium-magnesium phosphate and placebo, the second group received these minerals plus Glucophag and the third group received the minerals plus Efedrin 20 mg x 2. All the groups also received one month's treatment with Trijodthyronin - a thyreoidea hormone, 20 µg x 2. Over a period of three months the group receiving potassium-magnesium phosphate and efedrin steadily lost 12.5 kg and at the same time showed a significantly increased oxygen absorption per kilo. The group receiving potassium-phosphate achieved a steady weight loss of 8.6 kg and at the same time a significant increase on oxygen absorption and potassium throughout the body calculated per kilo. The group receiving minerals and Glucophag lost 8.0 kg with a slight increase in oxygen absorption per kilo and finally, the group receiving the placebo achieved a weight loss of 6.5 kg with a definite reduction in oxygen absorption and potassium throughout the body per kilo, see the following table. An increased skin temperature was noted in all groups during the experiment. All groups showed a marked drop in blood pressure, which was most pronounced for the group receiving Efedrin + potassium-phosphate. There were no side effects apart from the occasional upset stomach.

All previous experience with low-calorie and slimming diets have indicated a marked drop in oxygen absorption, both totally and per kilo of body weight, often related to the reduction in calorie percentage. It is rather unusual for a patient to show improved metabolism with increased oxygen absorption per kilo of body weight as well as a decisive reduction in weight, as in the experiments described above.

The above experiments with the addition of minerals (potassium 40 mmol, magnesium 17.5 mmol and phosphate 35 mmol) will decisively increase our understanding of the physiology of obesity and its treatment.

**TABLE**

| Group I n = 7 (K+, Mg²⁺, HPO₄²⁻) + placebo (1 tabl.x2) Food intake 1200–1400 kcal | Group II n = 7 (K+, Mg²⁺, HPO₄²⁻) + Efedrin 20 mg x2 | Group III n = 6 (K+, Mg²⁺, HPO₄²⁻) + Glucophag 500 mgx2 | Group IV n = 6 Placebo minerals + tabl. placebo |
|---|---|---|---|
| Initial weight 97,5 kg | Initial weight 100,5 kg | Initial weight 93,5 kg | Initial weight 97,0 kg |
| Weight after 12 weeks 87,9 kg (– 8,6 kg) | 88,0 kg (– 12,5 kg) | 85,5 kg (– 8,0 kg) | 90,5 kg (– 6,5 kg) |
| + Trijodthyronin 20 μg x 2 | | | |
| Weight after 16 weeks 84,9 kg | 85,0 kg | 83,5 kg | 86,5 kg |

X̄: Group I – III : 9,9 kg
X̄: Group IV : 6,5 kg

IV. The weight process in ob/ob-mice (spontaneously obese mice) receiving either traditional, highly salted pellet food or physiological food in the form of wholemeal flour.

On a diet of today's pellet fodder (R 3), containing 150 - 250 mmol sodium chloride per kilo, the genetically obese mouse species ob/ob-mice becomes grossly overweight after 6 weeks, quickly develops a pronounced resistance to insulin, and gradually develops diabetes of the same type as human-beings. After 3 - 4 months these mice are more than 100% overweight in comparison with control mice. In experiments with these mice they were fed a fixed quantity of calories of either R 3 or wholemeal flour or potato daily over extended periods of up to three months. The experiments show without a doubt that with isocaloric diet of either wholemeal flour or potato a very pronounced weight reduction is obtained for ob/ob-mice and a marked decrease in blood sugar. With an R 3 diet of 15 kilocalories a day, within four months the mice developed diabetes with blood sugar levels above 15 - 20 mmol/liter blood. This can be prevented entirely by feeding them on the physiological diet (wholemeal flour/potato) for up to three months.

## Claims

1. The use of a composition comprising potassium, magnesium, hydrogen phosphate or dihydrogen phosphate ions in an amount of 30 - 80 mmol $K^+$, 0 - 20 mmol $Mg^{2+}$ and 15 - 50 mmol $HPO_4^{2-}$ or $H_2PO_4^{1-}$ for the manufacture of a medicament for improving the cellular metabolism in human-beings with adequate calorie consumption in combination with a salt-deficient diet.

## Revendications

1. Usage pour la manufacture d'un médicament d'une composition comprenant des ions potassium, magnesium, hydrogénophosphate et dihydrogénophosphate, à dose de 30 - 80 mmoles de $K^+$, 0-20 mmoles de $Mg^{2+}$ et 15-50 mmoles de $HPO_4^{2-}$ or $H_2PO_4^-$ pour améliorer le métabolisme cellulaire des êtres humains avec une consommation correcte de calories combinée avec une diète déficiente en sel.

## Patentansprüche

1. Anwendung einer pharmazeutischen Zubereitung beinhaltend Kaliumionen, Magnesiumionen, Dihydrogenphosphationen- und Hydrogenphosphationen in einer Menge von 30-80 mmol $K^+$, 0-20 mmol $Mg^{2+}$, 15-50 mmol von $HPO_4^{2-}$ und 15-50 mmol $H_2PO_4^-$ zur Herstellung eines Arzneimittels zur Verbesserung des cellulären Metabolismus bei Menschen mit adäquatem Kalorienverbrauch in Kombination mit salzarmer Diät.

FIG.1

FIG. 2

FIG.3

FIG. 4